# EUROPEAN PATENT SPECIFICATION

(11) **EP 2 914 959 B1**
(45) Date of publication and mention of the grant of the patent: **27.06.2018**
(21) Application number: 12887735.4
(22) Date of filing: 01.11.2012
(51) Int. Cl.: G01N 29/04, G01N 29/12, B07C 5/14, G01N 33/46, G01N 22/00

(54) **METHOD AND SYSTEM FOR AUTOMATIC DETERMINATION OF TIMBER QUALITY IN FROZEN OR UNFROZEN CONDITION**
VERFAHREN UND SYSTEM ZUR AUTOMATISCHEN BESTIMMUNG EINER BAUMSTAMMQUALITÄT IN GEFRORENEM ODER UNGEFRORENEM ZUSTAND
PROCÉDÉ ET SYSTÈME DE DÉTERMINATION AUTOMATIQUE DE QUALITÉ DU BOIS À L'ÉTAT GELÉ OU NON GELÉ

(43) Date of publication of application: 09.09.2015
(73) Proprietor: RISE RESEARCH INSTITUTES OF SWEDEN AB, 501 15 Borås (SE)
(72) Inventor: LYCKEN, Anders, S-167 74 Bromma (SE); VIKBERG, Tommy, S-931 62 Skellefteå (SE)
(74) Representative: Platt, Timothy Nathaniel
(86) International application number: PCT/SE2012/051195
(87) International publication number: WO 2014/070057

(56) References cited:
- EP-A2- 1 707 955
- WO-A1-90/09578
- WO-A1-98/01737
- WO-A1-2006/016824
- WO-A1-2007/011296
- WO-A1-2007/011296
- GB-A- 2 440 555
- US-A- 4 514 680
- US-A1- 2003 146 767
- US-A1- 2003 218 468
- US-A1- 2003 222 658
- US-A1- 2005 216 226
- US-A1- 2010 295 556
- JULIAN MORENO CHAN ET AL: "Effects of moisture content and temperature on acoustic velocity and dynamic MOE of radiata pine sapwood boards", WOOD SCIENCE AND TECHNOLOGY ; JOURNAL OF THE INTERNATIONAL ACADEMY OF WOOD SCIENCE, SPRINGER, BERLIN, DE, vol. 45, no. 4, 22 June 2010 (2010-06-22), pages 609-626, XP019964945, ISSN: 1432-5225, DOI: 10.1007/S00226-010-0350-6

## Description

### Technical Field

The present invention relates to a method and Installation for automatic determination of the quality of wooden logs and sawn timber. Quality in this context refers to wood density, strength and/or modulus of elasticity. This determination is used, for example, in modern mills for automatic proper and accurate sorting of logs for various purposes and various sawlog products.

### Background Art

Resonance based technology is known and used for non-destructive quality classifying logs and boards. A hammer impulse against the end of a log for example generates acoustic waves in the log which are measured for various parameters such as velocity gradients, propagation, patterns, resonance frequency etc. Such acoustic impulse/resonance methods are described in US 6 782 732 (Huang et al.) and WO 2007/011296 A1 (Lindström), and are marketed for sawmill use by for example Fibre-Gen (Hitman LG 640) and A-sort AB.

Certain researchers have described the use of microwave scanning to estimate certain properties of a log such as density, moisture content or temperature. See for example "Microwave penetration in wood using imaging sensor", L. Hansson et al. Measurement 38 (2005) 15-20 and L Hansson et al "Finite element modeling (FEM) simulation of interactions between wood and microwaves", J Wood Sci (2006) DOI 10.1007/s10086-005-0794-8.

Winter use of microwave scanning of timber is dealt with in "How do microwaves respond to winter conditions?" a Technical Report submitted by Olle Hagman et al to the 58th Annual Meeting of the Forest Products Society, Grand Rapids Michigan 27-30 June 2004. It relates that the results of microwave scanning of sawn timber are skewed and unreliable when there is the possibility that a log or board or portion thereof is frozen requiring calibrations for each of those situations. Such calibrations are even impossible at around 0° C.

The acoustic/resonance based methods for determining log quality are simple and reliable as long as there is no chance that the logs or boards are frozen, completely or in the interior. Infrared measurement of temperature is only accurate as to the surface temperature. Frozen log portions give completely deceptive results, indicating a much higher log quality than is the case. A log which should have been sent to the chipper or to a low quality cutting pattern might be sent through the sawmill only to result in boards of bad quality.

It would be a great advantage to be able to make the automatic acoustic resonance quality determination reliable even for use when the logs or portions thereof may still be frozen when entering the sorting yard.

This is made possible by the method and installation according to the invention as disclosed in independent Claims 1 and 6.

### Brief Description of Drawings

The present invention will be described in the following with reference to a nonlimiting example, illustrated in the accompanying drawings, of which:
Fig.1 shows schematically an exemplary process in accordance with the invention whereby winter logs are quality sorted before breakdown.
Fig. 2 shows microwave scanning of pine timber at 3 different temperatures as well as transmission through air.
Fig. 3 shows acoustic resonance measurement of similar pine timber.
Fig. 4 is a plotting of the maximum acoustic frequencies for a large number of pieces of timber when frozen and unfrozen.

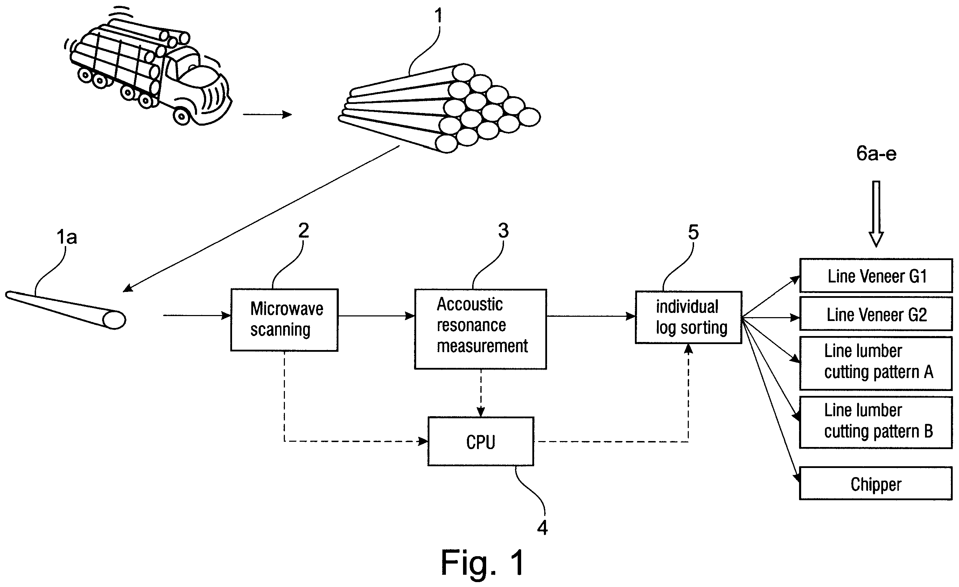

Accoustic resonance methods are used for example to sort logs before breakdown into different machine stress grades (MSG), for improved yield upon sawing, or into G1 and G2 veneer grades. Inaccurate sorting results in poorer sawmill yields. Fig. 1 shows how the system according to the invention could be set up for sorting prior to the individual logs entering the saw mill. An individual log 1a is taken from arriving logs 1 and is subjected to microwave scanning 2 followed by acoustic resonance measurement. It is also, of course, possible to perform these two steps in the reverse order. The results of the microwave scanning and the acoustic resonance measurement of the individual log are processed by computer means 4 and an instruction is sent to the log sorting station to direct the log to the proper line based on its quality, very low quality logs being sent to the chipper line to make biofuel, fiberboard or pulp.

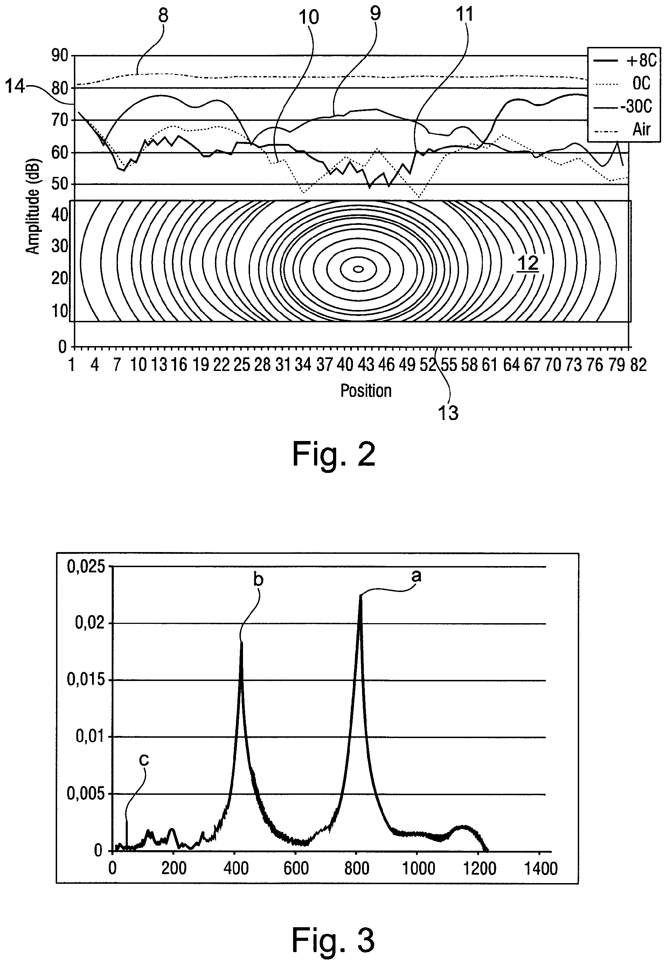

Existing sorting systems usually utilize only acoustic resonance measurement to determine log quality, a hammer impacts the end of the log and microphones record the frequencies and amplitudes of the resulting resonances. The frequency of greatest amplitude can be used as a good indication of the strength and density of the log, a higher resonant frequency corresponding to higher density. When looking at a sample reading shown in Fig. 3 for example, three harmonic peaks a, b, and c are registered with the maximum amplitude at peak a at a frequency of about 700 Hz, corresponding to a certain stiffness in the wood, indicating strength and quality. This measurement is used to automatically steer the log to the line where it will provide the best possible product yield either as veneer of different thicknesses, sawn boards from various cutting patterns or as chips.

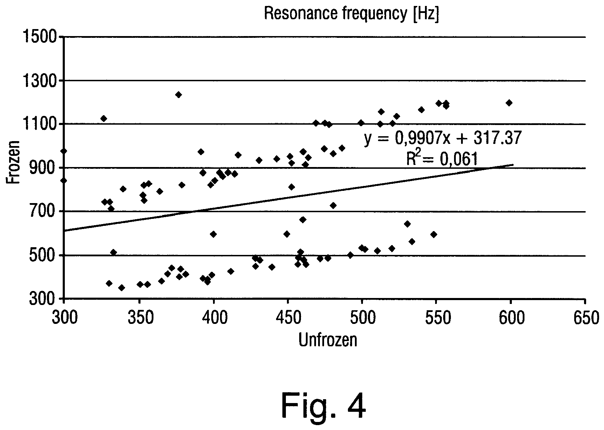

A problem with acoustic resonance measurement sorting arises when some of the logs may be frozen when measured. A frozen log often gives a frequency reading when frozen which is twice that of the same log when thawed. It is the frequency of greatest amplitude which is taken by the known system as indicative of log quality. Thus, when referring to Fig. 3, the frequency of 700 Hz would be taken as indicative of the log quality, even if the log were in a frozen state. This anomaly is shown in Fig. 4 which plots the highest resonant frequencies of some 50 logs both when frozen (the Y-axis) and when thawed (the X-axis). Most of the logs register double the frequency when frozen as when thawed (the relevant state for quality). All of the frozen logs registering the double frequency will be sent into the higher quality lines and ultimately produce sawn products which may have to be discarded because the strength of the wood is too low.

It has now been recognized according to the present invention that combining acoustic resonance measurement with microwave scanning may provide a way to more accurately sort logs even in a winter environment. According to the present invention, it is recognized that the microwave scanning of a log which is frozen either completely or only in the middle produces a typical pattern of attenuation. This is illustrated clearly in Fig. 2, showing a microwave scanning pattern across a piece of timber 12, the lateral position of the microwave reading in the timber being shown on the x-axis 13 and the amplitude of the signal being shown on the y-axis 14. The microwave pattern through air is the curve 8, which shows very little distortion. The curve 11 where the wood temperature is +8° C and the curve 10 where the wood temperature is 0° C display a typical attenuation dip for unfrozen wood. The curve for deep frozen wood at -30° C is labeled 9 and displays very little attenuation.

This difference in attenuation is exploited in the present invention which utilizes this phenomenon together with the usual acoustic resonance measurement to determine automatically when a log is frozen and discount the acoustic resonance reading thereof, indicating a high quality log.

Fig. 4 shows the effect of freezing on the acoustic resonance measurements for various qualities of logs. Experience makes it possible to classify logs receiving an acoustic measurement rating of below 600 Hz as being correctly measured and not having an incorrectly doubled value because it is frozen, since very few logs ever resonance measure in the unfrozen state below 300Hz. Logs having a resonance measurement of greater than 700 Hz can generally be assumed to have a doubled value, i.e. by being frozen. The problematical resonance values are in the 600- 700 Hz range, i.e. determining whether a particular log is a low quality log (for example 350 Hz when in the unfrozen state) or is a high quality log (700 Hz) which is not frozen. The present invention uses a supplementary microwave scanning and evaluation (significant attentuation=unfrozen, minimal attenuation=frozen) to determine whether the log is frozen and halve the acoustic resonance value if so.

The person skilled in the art will recognize that although the above described embodiment is directed to certain specified frequency ranges, these ranges may vary, but still within the claimed scope of the invention, depending on the type of wood measured impact method etc. These ranges may be determined by testing and calibration.

Although the example is described as being applied at a sawmill, the person skilled in the art will immediately understand that this method and system according to the invention can be applied with certain or all of the steps performed already in the woods and not at a sawmill.

The person skilled in the art will of course recognize that the present invention may be implemented in a variety of different manners within the scope of the claims, for example by utilizing the microwave attenuation result only when a certain range of frequencies are obtained from the acoustic resonance measurements and disregarding it for others. It is of course expected that feed-back information at the end of the sawmill line, i.e. actual quality evaluations will be utilized to further trim the sorting process to provide even better economy for winter operation.

## Claims

1. Method of non-destructive determination of wood quality, comprising subjecting an elongated wooden object (1a) to a non-destructive acoustic impulse/resonance measurement procedure (3) for structural properties such as strength or stiffness, generating a frequency spectrum having a resonance amplitude absolute maximum amongst all the harmonic frequencies in the spectrum, said absolute maximum being recorded as an indication of the structural quality of the wood, **characterized by** subjecting the wooden object (1a) to microwave scanning (2), registering the attenuation of the received microwaves across the wooden object, checking automatically in regions of the wooden object for increased microwave amplitude attenuation expected when in unfrozen condition, and in the absence of said increased microwave amplitude attenuation, determining that the log is frozen and taking as an indication of the structural quality of the wood a resonance amplitude reduced from said absolute maximum instead.

2. Method of non-destructive determination of wood quality according to claim 1, **characterized in that** said reduced resonance amplitude is an empirically determined fraction of said resonance amplitude absolute maximum.

3. Method of non-destructive determination of wood quality according to claim 1, **characterized in that** said reduced resonance amplitude taken is the resonance amplitude of a harmonic frequency of the frequency for the absolute maximum.

4. Method of non-destructive determination of wood quality according to claim 1, **characterized in that** a reduced resonance amplitude is only taken when the microwave amplitude attenuation registered during microwave scanning does not display a predetermined typical attenuation dip which indicates unfrozen wood.

5. System for sorting elongated wooden objects (1) by wood quality, comprising a measurement station (3) for subjecting the wooden objects (1) to a nondestructive acoustic impulse/resonance measurement procedure (3) for structural properties such as strength or stiffness, generating a frequency spectrum having a resonance amplitude absolute maximum (a) amongst all the harmonic frequencies (a, b, c) in the spectrum, said absolute maximum being recorded as an indication of the structural quality of the wood, and a sorting station (5) for automatic sorting of each elongated object coming from said and measurement station (3) and directing each elongated object to a line appropriate to the wood quality of the elongated object, **characterized by** the system further comprising a scanning station (2), having means for subjecting an elongated wooden object (1a) to microwave scanning, registering the attenuation of the received microwaves across the wooden object, and a central processing unit (CPU) (4), receiving signals from the scanning and measurement stations (2, 3) and controlling said sorting station (5), said CPU checking automatically in regions of the wooden object for increased microwave amplitude attenuation as expected when in unfrozen condition, and in the absence of said increased microwave amplitude attenuation, determining that the log is frozen and taking as an indication of the structural quality of the wood a resonance amplitude reduced from said absolute maximum instead.

6. System according to claim 5, **characterized in that** said scanning station is placed physically after said measurement station in the direction of flow of elongated wooden objects.

7. System according to claim 5, **characterized in that** one, several or all of said stations are located remote from a log breakdown station.

8. System according to claim 7, **characterized in that** the scanning or measurement station is a hand held portable unit.

9. System according to one of claims 5 - 8, **characterized in that** said elongated wooden object is a wooden log.

10. System according to one of claims 5 - 8, **characterized in that** said elongated wooden object is wooden board.

## Patentansprüche

1. Verfahren zum zerstörungsfreien Bestimmen von Holzqualität, umfassend Aussetzen eines länglichen hölzernen Objekts (1a) einem zerstörungsfreien akustischen Impuls-/Resonanzmessvorgang (3) für Struktureigenschaften, wie etwa Festigkeit oder Steifigkeit, Erzeugen eines Frequenzspektrums mit einem absoluten Resonanzamplitudenmaximum unter allen harmonischen Frequenzen in dem Spektrum, wobei das absolute Maximum als Indikator für die Strukturqualität des Holzes erfasst wird, **gekennzeichnet durch** Aussetzen des hölzernen Objekts (1a) einem Mikrowellenabtasten (2), Registrieren der Dämpfung der empfangenen Mikrowellen über dem hölzernen Objekt, automatisches Prüfen in Bereichen des hölzernen Objekts auf erhöhte Mikrowellenamplitudendämpfung, die bei einem ungefrorenen Zustand erwartet wird, und bei einem Nichtvorhandensein der erhöhten Mikrowellenamplitudendämpfung Bestimmen, dass der Stamm gefroren ist, und stattdessen Nehmen einer Resonanzamplitude, die gegenüber dem absoluten Maximum reduziert ist, als Indikator für die Strukturqualität des Holzes.

2. Verfahren zum zerstörungsfreien Bestimmen von Holzqualität nach Anspruch 1, **dadurch gekennzeichnet, dass** die reduzierte Resonanzamplitude ein empirisch bestimmter Anteil des absoluten Resonanzamplitudenmaximums ist.

3. Verfahren zum zerstörungsfreien Bestimmen von Holzqualität nach Anspruch 1, **dadurch gekennzeichnet, dass** die genommene reduzierte Resonanzamplitude die Resonanzamplitude einer harmonischen Frequenz der Frequenz für das absolute Maximum ist.

4. Verfahren zum zerstörungsfreien Bestimmen von Holzqualität nach Anspruch 1, **dadurch gekennzeichnet, dass** eine reduzierte Resonanzamplitude nur genommen wird, wenn die Mikrowellenamplitudendämpfung, die während eines Mikrowellenabtastens registriert wird, keine vorbestimmte typische Dämpfungssenkung anzeigt, die auf ungefrorenes Holz hinweist.

5. System zum Sortieren von länglichen hölzernen Objekten (1) nach Holzqualität, umfassend eine Messstation (3) zum Aussetzen der hölzernen Objekte (1) einem zerstörungsfreien akustischen Impuls-/Resonanzmessvorgang (3) für Struktureigenschaften, wie etwa Festigkeit oder Steifigkeit, Erzeugen eines Frequenzspektrums mit einem absoluten Resonanzamplitudenmaximum (a) unter allen harmonischen Frequenzen (a, b, c) in dem Spektrum, wobei das absolute Maximum als Indikator für die Strukturqualität des Holzes erfasst wird, und eine Sortierstation (5) zum automatischen Sortieren jedes länglichen Objekts, das von der Messstation (3) kommt, und Richten jedes länglichen Objekts zu einer Strecke, die für die Holzqualität des länglichen Objekts angemessen ist, **gekennzeichnet durch** das System, ferner umfassend eine Abtaststation (2) mit Mitteln zum Aussetzen eines länglichen hölzernen Objekts (1a) einem Mikrowellenabtasten, Registrieren der Dämpfung der empfangenen Mikrowellen über dem hölzernen Objekt, und eine zentrale Verarbeitungseinheit (CPU) (4), die Signale von der Abtast- und Messtation (2, 3) empfängt und die Sortierstation (5) steuert, wobei die CPU Bereiche des hölzernen Objekts automatisch auf erhöhte Mikrowellenamplitudendämpfung prüft, die bei einem gefrorenen Zustand erwartet wird, und bei einem Nichtvorhandensein der erhöhten Mikrowellenamplitudendämpfung bestimmt, dass der Stamm gefroren ist, und stattdessen eine Resonanzamplitude, die gegenüber dem absoluten Maximum reduziert ist, als Indikator für die Strukturqualität des Holzes nimmt.

6. System nach Anspruch 5, **dadurch gekennzeichnet, dass** die Abtaststation physisch nach der Messstation in der Bewegungsrichtung von länglichen hölzernen Objekten platziert ist.

7. System nach Anspruch 5, **dadurch gekennzeichnet, dass** sich eine, mehrere oder alle Stationen von der Stammzergliederungsstation entfernt befinden.

8. System nach Anspruch 7, **dadurch gekennzeichnet, dass** die Abtast- oder Messstation eine in der Hand gehaltene tragbare Einheit ist.

9. System nach einem der Ansprüche 5 - 8, **dadurch gekennzeichnet, dass** das längliche hölzerne Objekt ein hölzerner Stamm ist.

10. System nach einem der Ansprüche 5 - 8, **dadurch gekennzeichnet, dass** das längliche hölzerne Objekt ein hölzernes Brett ist.

## Revendications

1. Procédé de détermination non destructive de la qualité d'un bois, consistant à soumettre un objet en bois (1a) allongé à une procédure (3) non destructive de mesure d'impulsions / résonances acoustiques pour des propriétés structurelles telles que la résistance ou la raideur, générer un spectre de fréquences ayant un maximum absolu d'amplitude de résonance parmi toutes les fréquences harmoniques dans le spectre, ledit maximum absolu étant enregistré en tant qu'indication de la qualité structurale du bois, **caractérisé par** le fait de soumettre l'objet en bois (1a) à un balayage (2) de micro-ondes, d'enregistrer l'atténuation des micro-ondes reçues sur l'objet en bois, de vérifier automatiquement dans des zones de l'objet en bois une atténuation accrue de l'amplitude des micro-ondes attendue à l'état non gelé, et, en l'absence de ladite atténuation accrue de l'amplitude des micro-ondes, de déterminer que la grume est gelée et prendre à la place comme indication de la qualité structurale du bois une amplitude de résonance réduite dudit maximum absolu.

2. Procédé de détermination non destructive de la qualité d'un bois selon la revendication 1, **caractérisé en ce que** ladite amplitude de résonance réduite est une fraction déterminée empiriquement dudit maximum absolu d'amplitude de résonance.

3. Procédé de détermination non destructive de la qualité d'un bois selon la revendication 1, **caractérisé en ce que** ladite amplitude de résonance prise considérée est l'amplitude de résonance d'une fréquence harmonique de la fréquence pour le maximum absolu.

4. Procédé de détermination non destructive de la qualité d'un bois selon la revendication 1, **caractérisé en ce qu'**une amplitude de résonance réduite n'est prise que lorsque l'atténuation d'amplitude de micro-ondes enregistrée lors du balayage à micro-ondes ne montre pas une baisse d'atténuation typique prédéterminée qui indique un bois non gelé.

5. Système de tri d'objets en bois (1) allongés selon la qualité du bois, comprenant une station de mesure (3) pour soumettre les objets en bois (1) à une procédure (3) non destructive de mesure d'impulsions / résonances acoustiques pour des propriétés structurelles telles que la résistance ou la raideur, pour générer un spectre de fréquence ayant un maximum absolu (a) d'amplitude de résonance parmi toutes les fréquences harmoniques (a, b, c) dans le spectre, ledit maximum absolu étant enregistré comme étant une indication de la qualité structurale du bois, et une station de tri (5) pour le tri automatique de chaque objet allongé provenant de ladite station de mesure (3) et dirigeant chaque objet allongé vers une ligne appropriée à la qualité du bois de l'objet allongé, **caractérisé en ce que** le système comprend en outre une station de balayage (2), ayant des moyens pour soumettre un objet en bois allongé (1a) à un balayage de micro-ondes, pour enregistrer l'atténuation des micro-ondes reçues à travers l'objet en bois, et une unité de traitement centrale (CPU) (4), recevant des signaux provenant des stations de balayage et de mesure (2, 3) et commandant ladite station de tri (5), ladite CPU vérifiant automatiquement dans des zones de l'objet en bois une atténuation accrue de l'amplitude des micro-ondes telle qu'elle est attendue dans un état non gelé, et, en l'absence de ladite atténuation accrue d'amplitude des micro-ondes, déterminant que la grume est gelée et prenant à la place comme indication de la qualité structurale du bois une amplitude de résonance réduite depuis ledit maximum absolu.

6. Système selon la revendication 5, **caractérisé en ce que** ladite station de balayage est placée physiquement après ladite station de mesure dans la direction d'avancée des objets en bois allongés.

7. Système selon la revendication 5, **caractérisé en ce qu'**une, plusieurs ou toutes lesdites stations sont situées à distance d'une station d'abattage de grumes.

8. Système selon la revendication 7, **caractérisé en ce que** la station de balayage ou de mesure est une unité portative apte à être tenue à la main.

9. Système selon l'une des revendications 5 à 8, **caractérisé en ce que** ledit objet en bois allongé est une grume en bois.

10. Système selon l'une des revendications 5 à 8, **caractérisé en ce que** ledit objet en bois allongé est un panneau en bois.
